# EUROPEAN PATENT APPLICATION

(11) **EP 1 908 748 A1**
(43) Date of publication of application: **09.04.2008**
(21) Application number: 06020944.2
(22) Date of filing: 05.10.2006
(51) Int. Cl.: C07C 233/06, C07C 209/62, C07C 211/38

(54) **Process for the preparation of memantine and its hydrochloric acid salt form**

(71) Applicant: KRKA, 8501 Novo mesto (SI)
(72) Inventor: Tihi, Jaroslav, 8000 Novo mesto (SI); Meleh, Marija, 8350 Dolenjske Toplice (SI); Vrecer, Franc, 8531 Straza pri Novem mestu (SI); Zupet, Rok, 1211 Ljubljana (SI); Pucelj, Joze, 8000 Novo mesto (SI); Pelko, Mitja, 8000 Novo mesto (SI)
(74) Representative: Becker Kurig Straus

(57) **Abstract**

The present invention relates to the synthesis of 1-amino-3,5-dimethyladamantane ("memantine") and its hydrochloric acid salt form. In particular a new intermediate (N-chloro-acetylamino-3,5-dimethyladamantane) for the synthesis of memantine as well as a new process for the preparation of memantine and its hydrochloric acid salt form using said intermediate is disclosed.

## Description

### Field of the invention

The present invention relates to a novel process for the preparation of memantine and its hydrochloric acid salt form based on a novel intermediate compound (N-chloro-acetylamino-3,5-dimethyladamantane).

### Background of the invention

Memantine hydrochloride is an amantadine derivative agent providing good and persistent activation of central nervous N-methyl-D-aspartate (NMDA) receptors, and, thus, can be used in the treatment of Parkinson's disease and Alzheimer's disease. According to chemical terminology memantine is designated either as 1-amino-3,5-dimethyladamantane or 1,3-dimethyl-5-adamantamine or 1,3-dimethyl-5-aminoadamantane or 3,5-dimethyl-1-adamantylamine.

There is an increasing evidence that memory loss and dementia in Alzheimer's disease are related to malfunctioning of the signals that pass messages between the nerve cells in the brain. In particular, the excessive activity of a chemical called glutamate contributes to the symptoms of Alzheimer's, as well as the progression of this disease. Glutamate acts on the NMDA receptors that are found on nerve cells in the brain. These receptors and nerve cells are involved in transmitting nerve messages in the brain that are important in learning and memory. Reportedly, glutamate damages the nerve cells by excessively stimulating the NMDA receptors. Memantine works by blocking the NMDA receptors in the brain, blocking the excessive activity of glutamate, but allowing the normal activation of the NMDA receptors that occurs when the brain forms a memory. Memantine may therefore improve brain functioning in Alzheimer's disease, and may also block the glutamate activity that could cause further damage to the brain cells.

US 3,391,142 (Eli Lilly) describes a procedure for the production of the drugs, *inter alia* memantine hydrochloride as shown in formula I: 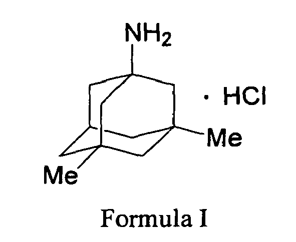 whereas 1,3-dimethyladamantane of formula IV: 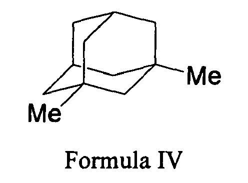 is brominated to obtain 1-bromo-3,5-dimethyladamantane of formula III, wherein X is Br: 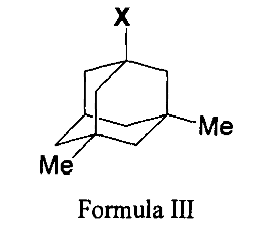 and further reacted to obtain memantine hydrochloride via alkaline hydrolysis of 1-acetamido-3,5,-dimethyladamantane of formula II:

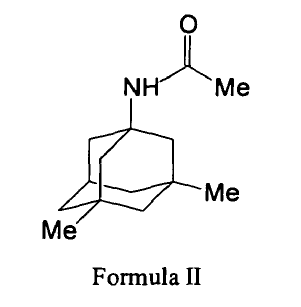

WO 2006/076562 (Teva) describes a similar process comprising reacting 1-halo-3,5-dimethyladamantane and nitrile in the presence of phosphoric acid.

US 4,122,193 (Merz) describes a process for the production of drugs, *inter alia* memantine hydrochloride, whereas 1-halogen-3,5-dialkyladamantane is reacted with urea via adamantyl urea derivate. An analogous approach via adamantyl urea derivate is applied in the Chinese application CN 1400205 (Guangzhou). Electrophilic substitution on metallated 3,5-dimethyladamantane by chloramine is disclosed in US 5,599,998 (Kraus).

WO 2005/062724 (Sun) describes a process for preparing memantine via hydroxyladamantane derivate of formula III wherein X is an OH group by bromating 1,3-dimethyl adamantane, hydrolysing and isolating 1-hydroxy-3,5-dimethyladamentane which is further converted to 1-acetamido-3,5,-dimethyladamantane and further to memantine.

Different crystalline forms of the memantine hydrochloric acid salt form are described in WO 2005/069742 (Sun) and WO 2006/076560 (Teva).

Caring for e.g. Alzheimer's disease and Parkinson's disease patients as well as a treatment/prevention of said and other related diseases results in high economic costs. Since memantine can be used in the treatment of Parkinson's disease and Alzheimer's disease, the search for new, economical processes for the preparation of memantine is an ongoing objective.

### Summary of the invention

The above problem has been solved by providing a novel process for the preparation of memantine and its hydrochloric acid salt form based on a novel intermediate compound (N-chloro-acetylamino-3,5-dimethyladamantane), which allows a rapid and economic preparation of the objective compound.

### Brief description of the drawings

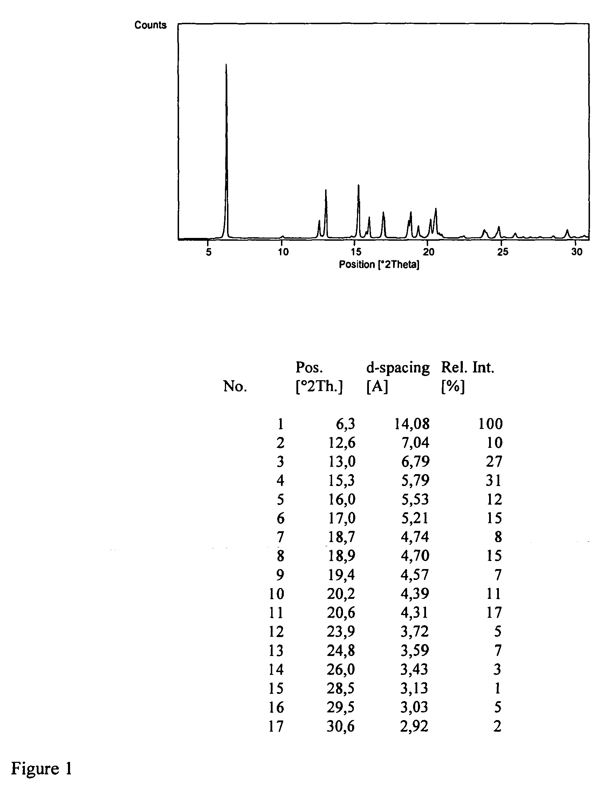

Figure 1 shows an X-ray powder diffraction of N-chloro-acetylamino-3,5-dimethyladamantane.

In a first aspect the present invention provides the compound N-chloro-acetylamino-3,5-dimethyladamantane. It has surprisingly been found out that the use of N-chloro-acetylamino-3,5-dimethyladamantane allows the production of memantine and/or its hydrochloride acid salt form at lower reaction temperatures, requires less reaction time, and allows the synthesis in solvents which are easy to regenerate.

The objective compound N-chloro-acetylamino-3,5-dimethyladamantane may be synthesized from 1-hydroxy-3,5-dimethyladamantane, 1-chloro and/or 1-bromo-3,5-dimethyladamantane which latter compound may be obtained according to techniques well known in the art, such is described e.g. in SU 1221866, WO 2005/062724, Russ. J. Org. Chem.; 29; 3.1; 1993; 454-456 or. Zh. Org. Khim.; 29;3;1993; 542-545 for 1-hydroxy-3,5-dimethyladamantane; 1-chloro-3,5-dimethyladamantane may be obtained according to techniques well known in the art such as is described e.g. CZ 284637; and 1-bromo-3,5-dimethyladamantane may be obtained according to techniques well known in the art such as is described e.g. J.Med.Chem, 6, 760, (1963) and EP 392059. 1-hydroxy-3,5-dimethyladainentane is reacted in chloracetonitrile in the presence of an acid. Such organic acid may be selected from the group consisting of hydrochloric acid, bromic acid, phosphorous acid and/or sulphuric acid; or organic acids selected from the group consisting of formic acid and/or acetic acid. In a preferred embodiment the reaction for preparing N-chloro-acetylamino-3,5-dimethyladamantane is carried out in the presence of chloroacetonitrile and sulphuric acid. In the most preferred embodiment the reaction for preparing N-chloro-acetylamino-3,5-dimethyladamantane is carried out in the presence of chloroacetonitrile, sulphuric acid and acetic acid.

N-chloro-acetylamino-3,5-dimethyladamantane of the present invention is characterized by a X-ray powder diffraction pattern exhibiting characteristic diffraction angles as shown in Fig. 1.

In a second aspect, the invention provides an improved process for the production of memantine and/or its hydrochloride acid salt form using the compound as detailed above. The process according to the present invention comprises the steps of reacting N-chloroacetylamino-3,5-dimethyladamantane with 1,1-diamino-synthone and/or 1,2-diamino-synthone in the presence of an organic solvent and optionally recovering memantine and/or its hydrochloride acid salt form.

The reaction partners 1,1-diamino synthone and/or 1,2-diamino synthone may be selected from the group comprising urea, thiourea, guanidine, 1,2-diamino ethane, o-phenylenediamine. According to a preferred embodiment they are selected from urea and/or thiourea.

The organic solvent may be selected from organic solvents, such as polar and water miscible solvents. Preferably solvents may be selected from the group consisting alcohol, which is preferably selected from C1-C5 alcohol and/or mixtures thereof. According to a more preferred embodiment alcohol is selected from methanol, ethanol and/or isopropanol.

The reaction is carried out for a time period and at a temperature sufficient to bring the reaction to an end, i.e. to obtain the desired compound. Preferably, the reaction is conducted for a time period of about 10 - 50 hours, more preferably from about 20 - 40, even more preferred about 24 - 30 hours, all at elevated temperatures of about 30 - 100 °C, more preferred 40 - 80 °C, even more preferred 50 - 80 °C, most preferred at the reflux temperature of the selected solvent. If desired the system may be set under pressure to increase the reflux temperature of the solvent selected.

After having performed the reaction for the given time period at the particular temperature the product obtained may be recovered as memantine and/or as its hydrochloride acid salt form. In a preferred embodiment it is recovered as memantine hydrochloride acid salt.

In principle, any known technique for recovering an organic compound from a reaction mixture may be applied. It has been found that good results may be obtained when performing the following steps (a) to (f).

In a first step (a) the reaction mixture is cooled to ambient temperature which may be affected by addition of water at the temperature lower than the temperature of reaction mixture and further by actively cooling the reaction vessel or leaving the reaction vessel without heating for a time sufficient, so reach the ambient temperature.

In a subsequent step (b), a base may be added to obtain an organic phase containing memantine. The base may be any suitable base, such as inorganic bases, e.g. NaOH or KOH, or organic bases, e.g. amines.

In step (c) an organic solvent is added, either to the reaction mixture as such or to the filtrate obtained in optional step (a), supra and mixed and treated, so as to transfer memantine to the new organic phase. The organic solvent is selected such that its polarity is lower as compared to the solvent used for the reaction. Non limiting examples for such organic solvents are acetates, ethers, higher alcohols, ketones, toluene and methylenchloride. An acetate may be selected from the group comprising ethyl acetate, isopropyl acetate, butyl acetate; an ether may be selected from the group consisting of diethylether, t-butylmethylether or diisopropylether; a higher alcohol may be selected from the group consisting of linear or branched C₄ - C₈ alcohols, such as e.g. butanol or isoamylalcohol; a ketone is e.g. acetone, diethylketone, or methylethylketone, etc., and mixtures thereof.

In a next step (d) the organic solvent phase containing memantine is separated, which may be simply effected by suction or decanting.

Optionally, step (c) and (d) may be repeated to ensure that essentially all of the desired product is transferred to the new organic solvent, which is pooled and further processed.

The organic phase thus obtained may then be treated (step(e)) to effect precipitation of the desired compound memantine hydrochlorid acid salt form, wherein memantine may be transformed to memantine hydrochloride acid salt form by adding hydrochloric acid. This may be further achieved by cooling the organic phase, e.g. to a temperature in the range of about -10 °C to 30 °C or by concentrating the organic phase, e.g. by evaporating the solvent, such as by heating and subsequent cooling and/or applying reduced pressure.

The precipitate is removed (step (f) from the solvent according to well known techniques, such as filtering, and the obtained memantine and/or memantine hydrochloride acid salt form may be dried, which may be performed at a temperature of between about 10 and 60 °C, more preferably between 35 and 45 °C. Drying may be achieved e.g. via application of heat, preferably carried out under ambient or reduced pressure or via contacting memantine and/or memantine hydrochloride acid salt form with humid air in a fluidized bed drier, wherein the atmosphere in the fluidized bed drier is at least 15% humidity. Preferably, vacuum drying in a commercially available vacuum dryer is applied.

Different methods for detecting the water content known to the skilled person may be used such as the method according to Karl Fischer, which is described inter alia in the European Pharmacopoeia 5th edition 2006; Method 2.5.12, which document is incorporated herein by way of reference.

According to a preferred embodiment of the present invention the obtained dried memantine hydrochloride acid salt form may be further recrystalized by dissolving the memantine hydrochloride acid salt form obtained in an organic solvent, such as C₁-C₅ alcohols, such as methanol, ethanol, propanol, butanol or pentanol, water, and mixtures thereof, and adding an "anti-solvent", i.e. a solvent with a lower polarity, which may be selected from the group as detailed for step (b), supra.

In the present invention the memantine hydrochloride acid salt form has a particle size such that average particle size is in the range of 3 to 150 µm, preferably 5 to 50 µm, more preferably 10 to 30 µm.

In order to determine the particle size, the particles to be subjected to the particle size measurement are first suspended in isoparafinic oil (Isopar^{®}) and then subjected to a size determination in a Malvern Mastersizer MS 2000 instrument. Usually, 100-800 mg of substance is dispersed in 5-8 ml of oil. According to manufacturer's information, the Malvern Mastersizers allow for a measurement of particle size distributions in the range of 20 nm to 2000 µm, with a precision of better than 0.5%. Particle sizes are determined by measuring the angular distribution of laser light scattered by a homogeneous suspension of particles. The size distribution is determined from the light scattering data using the theory of light scattering developed by Gustav Mie.

A GC method is used to determine an amount and purity of N-chloro-acetylamin-3,5-dimethyladamantane, 1-acetamido-3,5-dimethyladamantane and memantine hydrochlorid acid salt form. The tests are carried out in Capillary, HP-5 (30 m x 0.32 mm, film thickness 0.25 µm) column. The chromatograph is equipped with a FID detector set at 250°C; Injector temperature 210 °C; and with helium as a carrier gas.

According to a preferred embodiment of the present invention, memantine and/or memantine hydrochloride acid salt form obtained by the process according to the present invention may be used *inter alia* for the preparation of a pharmaceutical composition for the prevention and/or treatment of Alzheimer's disease, Parkinsonism, cerebral and/or peripheral spasticity, neuropathic pain, AIDS-related dementia and organic brain syndrome.

The pharmaceutical compositions may be in a solid or liquid dosage form. Exemplary solid dosage forms include tablets, sachets, lozenges, powders, pills, pellets, or granules. The solid dosage form may be, for example, immediate release dosage form, a fast melt dosage form, orally disintegrating dosage form, modified release dosage form, lyophilized dosage form, delayed release dosage form, extended dosage form, pulsatile dosage form, mixed immediate and modified release dosage form, or combinations thereof. A solid dosage tablet formulation is preferred. The solid dosage form is preferably an immediate release dosage form offering advantages regarding the bioavailability of the active compound.

The tablet dosage form is either in the form of film coated tablets or in the form of tablets which disintegrate very fast, *i.e.* in less than 2 minutes in oral cavity releasing particles including drug and does not enable the release of drug in the mouth. Film coated tablets are manufactured by coating the tablet cores produced by direct compression or via granulation step with a dispersion containing at least one polymer which prevents drug dissolution upon contact of tablet with saliva and resulting unpleasant mouth filling and at least one excipient chosen from pigments, antitacking agents, plasticizers.

Tablet cores may be produced either via granulation step or by direct compression of active ingredient together with at least one pharmaceutical excipient chosen from
- fillers comprising lactose in anhydrous or hydrated form such as lactose monohydrate, microcrystalline cellulose, siliconised microcrystalline cellulose, starch, powdered cellulose, sugar derivatives such as manitol, sorbitol, lactitol, saharose, earth alkali phosphates such as calcium phosphate or mixtures thereof,
- binders comprising polyvinyl pyrolidone, cellulose ethers such as hydroxypropyl cellulose, hydroxypropylmethyl cellulose, hydroxyethyl cellulose, microcrystalline cellulose, starch, pregelatinised starch or mixtures thereof,
- disintegrants comprising starch derivatives such as corn starch and pregelatinised starch, low substituded hydroxypropyl cellulose, microcrystalline cellulose, carboxymethyl cellulose salts such as calcium and sodium salts, polacrylin potassium, crospovidone, sodium starch glycolate or mixtures thereof;
- wetting agents such as sorbitan derivatives, sodium dodecyl sulphate, poloxamere or the mixture thereof,
- lubricants such as sodium staryl fumarate, metal stearates such as magnesium, calcium, aluminium stearate, hydrogenated oils such as hydrogenated vegetable or castor oil, talc, colloidal silicon dioxyde or their mixtures,
- in case modified release of drug is required, controlled release polymers such as swellable cellulose ethers represented but not limited by hydroxypropylmethyl cellulose, hydroxypropyl cellulose which exhibit viscosity of 100 or more mPas at room temperature in 2% w/w dispersion in water, ethylcellulose, methacrylic ester copolymers, ammonia methacrylate copolymers, natural sacharides such as xanthan gum, alginic acid salts, carragenans or the mixture thereof are incorporated in the tablet cores.

The granulation can be performed either using solvents (wet granulation) or dry granulation. Water or water miscible organic solvents can be used in wet granulation step using state of the art granulation equipment such as fluid bed granulators, high or low shear mixers. The obtained granulate can be further dried by using microwave, fluid bed or convection driers (drying chambers with or without using under pressure or vacuum). Dry granulation can be performed by using compactors or briquetting.

As an alternative to granulation, a blended composition may be compressed directly into a compacted dosage form using direct compression techniques. Direct compression produces a more uniform tablet without granules. The proper use of these and other excipients in direct compression tableting is known to those skilled in the art with experience and skill in particular formulation challenges of direct compression tableting.

In a preferred embodiment the direct compression can be used to prepare tablet cores.

Tablet cores can be further coated in order to mask the taste of active ingredient and/or excipients. Taste masking can be achieved by using polymers which are soluble in saliva such as hydroxypropylmethyl cellulose, hydroxypropyl cellulose, methyl cellulose, enteric coating polymers such as methacrylic acid copolymers (Eudragit L types), cellulose acetate phthalate, cellulose acetate succinate, hydroxypropyl methylcellulose phthalate and/or theirs soluble salts, polyvinyl alcohol or insoluble polymers, such as ethyl cellulose or aminoalkyl methacrylate copolymers copolymers (Eudragit E, Eudragit RL and/or Eudragit RS types), and mixtures thereof.

Additional excipients can be used in forming film coat comprising antitacking and antistatic agents such as talc, magnesium stearate, pigments or coloring agents, plasticizers such as polyethylene glycol or the mixture thereof. Coating can be performed in state of the art coating equipment such as perforated coating pans, fluid bed coaters etc..

In another embodiment memantine and/or memantine hydrochloride acid salt form obtained by the present process can be used for the preparation of orally disintegrating tablets.

Orally disintegrating tablets can be produced according the process comprising the following steps: preparing granules, mixing the granules with active ingredient and excipients and compressing the obtained mixture to the desired form. Memantine and/or memantine hydrochloride acid salt form can be included in granules.

In another aspect of the invention of orally disintegrating tablets which are more acceptable for patients experiencing difficulties with swallowing were based on incorporation of coated particles of drug substance into the tablets. Coated particles can be obtained either by direct coating of drug particles with coating dispersion containing polymers, which prevent dissolution of drug particles upon disintegration of tablet in oral cavity, and other pharmaceutical acceptable excipients selected from state of the art known plasticizers, antitacking agents, solubilisizers. Coating can be performed using state of the art coating equipment such as top, bottom and tangential spray apparatus. In another embodiment of the invention coated particles can be obtained by coating of neutral cores, such as sugar or microcrystalline cellulose pellets with a dispersion which can be in the form of a solution, suspension or emulsion of drug substance and other excipients selected from fillers (saccharose, lactose, manitol, xylitol, sorbitol), binders (water soluble polymers such as methyl cellulose, hypromelose, hyprolose, hydroethyl cellusoe, povidone), wetting agents such as sorbitan derivatives, sodium dodecyl sulphate, poloxamere or the mixture thereof and/or antitacking agents such as talc and/or glycerol monostearate. Obtained particles are further coated with film coating which prevents release of active drug from coated particles upon disintegration of tablets in oral cavity. Such coating is based on saliva insoluble polymers such as ethyl cellulose or aminoalkyl methacrylate copolymers (Eudragit E, Eudragit RL and/or Eudragit RS types) and optionally other excipients selected form plasticizers, antitacking and wetting agents. Plasticizers are used in amount of 1-50 % calculated on the weight of the taste masking polymer used what prevents damages of the film coating during the compression of coated particles together with other excipients into tablets, what would result in partial dissolution of active drug in saliva and consequently bad taste.

Obtained coated particles are in the next step mixed with from state of the art known excipients for producing orally disintegrating dosage forms selected from fillers (such as manitol, sorbitol, xylitol, lactose), disintegrating agents (corn starch and pregelatinised starch, low substituted hydroxypropyl cellulose, microcrystalline cellulose, carboxymethyl cellulose salts such as calcium and sodium salts, polacrylin potassium, crospovidone, sodium starch glycolate or the mixture thereof), sweeteners (acesulfam K, sucralose, alitame, aspartame, saccharin sodium, dipotasium glycyrrhizinate, stevia and/or thaumatin and the mixtures thereof), flavoring agents( such as natural or synthetic aromas), lubricants (such as magnesium, calcium or aluminium stearate, magnesium lauryl stearate, sodium stearyl fumarate, fatty acid such as stearic acid, or hydrogenated vegetable oil or mixtures thereof). Organic acids (such as citric, tartaric, malic, fumaric acid) can be optionally added to the tabletting mixture to improve the mouth filling upon disintegration of tablets in the oral cavity.

The active drug is released from particles upon dissolution of polymer coating or through the pores obtained upon contact of coated particles with physiological fluids.

*Preparation of granules:* Mannitol, microcrystalline cellulose, low-substituted hydroxypropyl cellulose (HPC), aspartame and cross-linked polyvinyl pyrrolidone (crospovidone) can be produced by mixing in a high shear mixer and granulated with purified water. The composition of the granules can be as follows:

| 1. Granules | |
|---|---|
| 1.1. Mannitol | 60 - 80 wt. % |
| 1.2. Microcrystalline cellulose | 5-15 wt. % |
| 1.3. Low-substituted HPC | 5 - 20 wt. % |
| 1.4. Memantine HCI | 10 - 20 wt. % |
| 1.5 Crospovidone | 5 - 20 wt. % |

*Preparation of ODT:* Obtained granulate may be produced by drying (Loss on drying preferably less than 2 %, most preferably less than 1.5 %). The dried granulate can be further coated with polymer insoluble in saliva dispersion such as aminoalkyl methacrylate copolymer water based dispersion (Eudragit EPO) containing also at least one excipients chose from wetting agent such as sodium lauryl sulphate, antitacking agent such as stearic acid, magnesium stearate, talc, plasticizer; subsequently mixed with the sweetener and calcium silicate. Finally, magnesium stearate can be admixed and the mixture was compressed to tablets at < 60 N. The disintegration times of the obtained tablets were below 30 s. They were measured in purified water according to the method described in Ph. Eur. 07/2005:0478 ; Chapter 2.9.1. Test A

The preferred composition of the ODT comprising granules from the first step can be as follows:

| **1. Granules** | **2. Active Ingredient** | **3. Calcium silicate** | **4. Magnesium stearate** | **5. Purified water** |
|---|---|---|---|---|
| 50 - 80 wt. % | 1 - 20 wt. % | 5 - 25 wt. % | 0.5 - 5 wt. % | As needed |

Different types of mannitol can be used, such as spray-dried mannitol, mannitol for direct compression and conventional mannitol. The average particle size of the mannitol may be within a range of from 10 to 500 µm, preferably 100 to 200 µm.

Different types of microcrystalline cellulose can be used for preparing granules, such as for example Avicel PH 101 and Avicel PH 102 or other commercially available microcrystalline celluloses. Besides the function of diluent, it has also binding, lubricating and disintegrating properties.

The low-substituted HPC may be selected from the group with a content of hydroxypropyl groups of 7.0 to 12.9 % by weight.

Aspartame may be used as a preferred sweetener. Examples of sweeteners are acesulfame K, sucralose, alitame, saccharin sodium, dipotassium glycyrrhizinate, stevia, and thaumatin.

The calcium silicate may be in a crystalline form, amorphous form or used as a mixture thereof. The average particle size of calcium silicate may be in the range of from 1 to 500 µm, preferably 1 to 100 µm. The most preferred is RxCipient FM 1000.

Various types of punches may be used for compressing tablets such as flat, biconvex, bevel edged, round, oval or capsule shaped and others. Friability of the tablets is preferably less than 1 %.
The following examples illustrate the invention without limiting it thereto.

### Examples

X-ray powder diffraction patterns were obtained by a Phillips PW3040/60 X'Pert PRO diffractometer using CuK_{α} radiation.

FT-IR spectra were recorded on a FT-IR spectrometer Spectrum 100 and System GX Perkin Elmer at a resolution of 4 cm⁻¹.

### Example 1 (via N-chloro-acetylamino-3,5-dimethyladamantane route)

### Preparation of N-chloro-acetylamino-3,5-dimethyladamantane

An amount of 12 g of 1-hydroxy-3,5-dimethyladamantane was placed in a round bottom flask together with 20 ml of chloroacetonitrile and 5 ml of acetic acid and heated to 50 °C. 18 ml of concentrated sulphuric acid was added drop wise over a period of about 1.5 hour while maintaining the temperature at about 50 °C. After the addition of sulphuric acid the reaction mixture was stirred for another 2 hours at the same temperature.

The reaction mixture was then slowly added to 200 ml of cold water with a temperature between 0 and 5 °C while intensive stirring and the mixture obtained was stirred for about 0.5 hour at a temperature of 10 °C, then filtered and rinsed with demineralised water.

The resulting product was suspended in 100 ml of demineralised water at ambient temperature, stirred for another 45 minutes, filtered and rinsed with demineralised water.

Finally, the product was dried in an air drier for about 6 hours at the 50°C until a constant weight was obtained. An amount of 15.51 g of N-chloro-acetylamino-3,5-dimethyladamantane was obtained, corresponding to a yield of 91 %.

### Preparation of memantine HCl

An amount of 5 g of N-chloro-acetylamino-3,5-dimethyladamantane, 1.55 g of thiourea and 25 ml of isoproanol was heated at the reflux temperature (below 85°C) for 16 hours. After the reaction was completed the reaction mixture was cooled to ambient temperature. An amount of 65 ml of demineralised water was added to the obtained suspension and mixed for about 0.5 hour. The precipitate was filtered to remove side products, such as thiohydantoine, and rinsed with 10 ml of demineralised water.

20 ml of isopropyl acetate have been added to filtrate, followed by addition 4.5 ml of 5 M NaOH while stirring.

The isopropyl acetate phase was separated and the rest of the reaction mixture was reextracted with another 50 ml of isopropyl acetate. The first and the second isopropyl acetate phases were mixed, rinsed with 10 ml of demineralised water and concentrated to 25 ml under vacuum. The solution was cooled to temperature between 0 to -10 °C and 1.8 ml of concentrated HCl was added drop wise while stirring. Reaction mixture was stirred for about 1 hour, filtered, rinsed with isopropyl acetate and finally dried in a vacuum drier for 4 hours at 40 °C. An amount of 3.2 g of memantine HCl was obtained, corresponding to a yield of 76%.

### Preparation of crystal memantine HCl

An amount of 3 g of memantine HCl as obtained in Example 1 was dissolved in 7.2 ml of methanol at 50 °C. After the memantine HCl was completely dissolved a 40 ml of acetone was added drop wise in a period of 1 hour and than the mixture was slowly cooled to the temperature of-10 °C, stirred for about 1 hour, filtered, rinsed with 5 ml of chilled acetone and finally dried in a vacuum drier for 3 hours at 50 °C. An amount of 2,4 g of memantine HCl of the crystalline form I as defined in WO 2006/076562 was obtained, corresponding to a yield of 80 %. The purity of the product was 99,94 %, with a maximal impurity of 0.02 %, 1-acetamido-3,5-dimethyladamantane not detected.

Total yield of the process was 55 %.

### Example 2

Composition of tablet cores obtained by direct compression:

| | | *E1* | *E2* | *E3* | *E4* | *E5* |
|---|---|---|---|---|---|---|
| 1 | Memantine HCl | 10.0 mg | 10.0 mg | 5.0 mg | 10.0 mg | 10.0 mg |
| 2 | Lactose monohydrate | 63 mg | / | 68.0 mg | 144.5 mg | 140.0 mg |
| 3 | Croscarmellose sodium | / | 63 mg | 6.0 mg | 12.0 mg | 10.0 mg |
| 4 | Microcrystalline cellulose | 21 mg | 21 mg | 35.0 mg | 60.0 mg | 87.5 mg |
| 5 | Colloidal silicon dioxide | 1mg | 1mg | 1.0 mg | 2.0 mg | / |
| 6 | Talc | 4.5 mg | 4.0 mg | 4.5 mg | 10.0 mg | / |
| 7 | Magnesium stearate | 1 mg | 1 mg | 0.5 mg | 1.5 mg | 2.5 mg |
| | Weight of tablet cores | 100.5 mg | 100.0 mg | 120.0 mg | 240.0 mg | 245.0 mg |

Active ingredient was premixed with part of lactose monohydrate (10 % (w/w)) for 10 minutes. The rest of lactose, crosscarmelose sodium and microcrystalline cellulose were added to the premixture and blended for another 10 minutes. Talc, colloidal silicon dioxide and magnesium stearate were admixed at the end to the obtained mixture.

Composition of tablet cores obtained via wet granulation:

| | *E6* | *E7* | *E8* |
|---|---|---|---|
| Memantine HCl | 10.0 mg | 10.0 mg | 10.0 mg |
| hypromelose | 2.0 mg | / | / |
| hyprolose | / | 3.0 mg | / |
| povidone | / | / | 2.0 mg |
| Lactose monohydrate | 135.0 mg | 160.0 mg | 120.0 mg |
| Sodium starch glycolate | | 10.0 mg | |
| Croscarmellose sodium | 10.0 mg | / | 6.0 mg |
| Microcrystalline cellulose | 85.5 mg | 52.6 mg | 50.0 mg |
| Corn starch | / | / | 56.0 mg |
| Colloidal silicon dioxide | / | 2 mg | 1.0 mg |
| Talc | / | / | 2.5 mg |
| Magnesium stearate | 2.5 mg | 2.4 mg | 2.5 mg |
| Weight of tablet cores | 245.0 mg | 240.0 mg | 240.0 mg |

Drug, lactose monohydrate, part of microcrystalline cellulose and part of disintegrant were granulated with 4 % water solution of binder. The obtained granulate was dried, sieved and mixed with the rest of ingredients and compressed into tablet cores.

Composition of film coating for the coating of tablet cores obtained under examples E1-E8

| | **A (mg)** | **B (mg)** | **C (mg)** | **D (mg)** | **E(mg)** | **F(mg)** |
|---|---|---|---|---|---|---|
| hypromellose | / | / | 7.0 | 3.5 | 3.0 | / |
| polyvinyl alcohol / | | / | / | / | / | 3.0 |
| methacrylic acid - ethyl acrylate copolymer (1:1)* | 3.0 | 2.0 | / | / | / | / |
| titanium dioxide | 0.5 | 0.15 | 0.3 | 0.25 | 0.25 | 1.0 |
| triacetin | 0.3 | / | / | / | / | / |
| Macrogo16000 | / | 0.2 | / | / | / | 0.5 |
| Macrogo1400 | / | / | 0.08 | 0.05 | / | 0.5 |
| Propylene glycole | / | / | / | / | 0.4 | / |
| iron oxide black | / | 0.02 | 0.02 | / | 0.05 | / |
| simethicone emulsion | 0.02 | 0.03 | / | / | / | / |
| talc | 1.18 | 0.8 | 2.6 | 1.2 | 1.3 | 1.0 |
| Weight of film coating/tablet core | 5.0 | 3.2 | 10.0 | 5.0 | 5.0 | 6.0 |

| | | | | | | |
|---|---|---|---|---|---|---|
| *... Commercial dispersion of Eudragit® L30D is used, which contain also sodium lauryl sulphate and polysorbate 80 | | | | | | |

Examples of orally disintegrating formulations:

### Example 3

| Composition of granulate: | |
|---|---|
| 1) Memantine HCl | 300.0 g |
| 2) Lactose monohydrate | 1,500.0 g |
| 3) Crospovidone | 160.0 g |
| 4) Hypromellose 606 | 40.0 g |

Hypromelose was dissolved in purified water to obtain 4 w/w % concentration and is sprayed onto mixture of ingredients under 1-3 in fluid bed granulating equipment GPCG-3. The dried granulate is further coated with the dispersion described below.

| | |
|---|---|
| Granules | 2,000.0 g |
| Eudragit E PO | 20.0 g |
| Sodium lauryl sulphate | 2.0 g |
| Stearic acid | 3.0 g |
| Magnesium stearate | 5.0 g |
| Purified water ad | 200.0 g |

### Preparation of compression mixture:

1015.0 g of obtained coated granulate is mixed with manitol (500.0 g) microcrystalline cellulose 101 (200.0 g), crospovidon (200.0 g) acesulfam potassium (10.0 g), flavour 25.0 g, magnesium stearate 12.0 g in appropriate mixer and compressed into tablets containing 10 mg of memantine HCl each.

### Example 4

| Step A) | |
|---|---|
| Composition of pellet cores: | |
| Microcrystalline pellets (250-350 µm) | 1500,0 g |
| Memantine HCl | 200.0 g |
| Hypromelose 603 | 10.0 g |
| Lactose | 240.0 g |
| Talc | 50.0 g |

Microcrystalline cellulose pellets are coated by spraying the dispersion obtained by dispersing the rest of ingredients in purified water in Wurster type GPCG-3. The coated pellets were dried in the coating equipment and further coated with taste masking coating in step B composed from:

| | |
|---|---|
| 1. Eudragit E PO | 300,0 g |
| 2. Sodium lauryl sulfate | 30.0 g |
| 3. Stearic acid | 45.0 g |
| 4. Talc | 140.0 g |
| 5. Pigment | 140.0 g |

Separately are prepared dispersions of ingredients under 1-3 in purified water with concentration of solid in dispersion 14% and dispersion of talc and pigment in water. Both dispersions are mixed and sprayed onto pellets obtained under step A.

The dried pellets obtained in step B are mixed with excipients as described in example 9 under Preparation of compression mixture and compressed into tablets each containing 5 mg of memantine HCl.

### Example 5

| Step A) Composition of pellet cores: | |
|---|---|
| Sugar spheres (300-400 µm) | 1500,0 g |
| Memantine HCl | 200.0 g |
| Hypromelose 603 | 10.0 g |
| Lactose | 240.0 g |
| Talc | 50.0 g |

Microcrystalline cellulose pellets are coated by spraying the dispersion obtained by dispersing the rest of ingredients in purified water in Wurster type GPCG-3. The coated pellets were dried in the coating equipment and further coated with taste masking coating in step B composed from:

| | |
|---|---|
| 1. Eudragit L30D | 150,0 g |
| 2. Macrogol 6000 | 50.0 g |
| 3. polysorbate 80 | 4.0 g |
| 4. Talc | 100.0 g |
| 5. Pigment | 100.0 g |

Separately are prepared dispersions of ingredients under 1-3 in purified water and dispersion of talc and pigment in water. Both dispersions are gently mixed and sprayed onto pellets obtained under step A.

The dried pellets obtained in step B are mixed with excipients as described in example 9 under Preparation of compression mixture and compressed into tablets each containing 10 mg of memantine HCl.

## Claims

**1.** N-chloro-acetylamino-3,5-dimethyladamantane according to formula II b

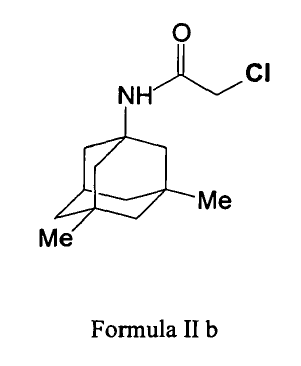

**2.** The compound according to claim 1, **characterized by** a X-ray powder diffraction pattern exhibiting characteristic diffraction angels at
| No. | Pos. [°2Th.] | d-spacing [A] | Rel. Int. [%] |
|---|---|---|---|
| 1 | 6,3 | 14,08 | 100 |
| 2 | 12,6 | 7,04 | 10 |
| 3 | 13,0 | 6,79 | 27 |
| 4 | 15,3 | 5,79 | 31 |
| 5 | 16,0 | 5,53 | 12 |
| 6 | 17,0 | 5,21 | 15 |
| 7 | 18,7 | 4,74 | 8 |
| 8 | 18,9 | 4,70 | 15 |
| 9 | 19,4 | 4,57 | 7 |
| 10 | 20,2 | 4,39 | 11 |
| 11 | 20,6 | 4,31 | 17 |
| 12 | 23,9 | 3,72 | 5 |
| 13 | 24,8 | 3,59 | 7 |
| 14 | 26,0 | 3,43 | 3 |
| 15 | 28,5 | 3,13 | 1 |
| 16 | 29,5 | 3,03 | 5 |
| 17 | 30,6 | 2,92 | 2 |
± 0.2° Theta.

**3.** A process for preparing memantine and/or its hydrochloride acid salt form comprising the steps of:
a.) reacting of Formula III, wherein X is Br, Cl or OH group to obtain a compound of Formula II b i.e. N-chloro-acetyamino-3,5-dimethyladamantane.
b.) reacting N-chloro-acetyamino-3,5-dimethyladamantane according to claim 1 or 2 with 1,1-diamino synthone and/or 1,2-diamino synthone in the presence of an organic solvent, and optionally recovering memantine and/or its hydrochloride acid salt form from reaction mixture.

**4.** The process according to claim 3, wherein the 1,1-diamino synthone and/or 1,2-diamino synthone is selected from urea, thiourea, guanidine, 1,2-diamino ethane, o-phenylenediamine, preferably from urea and/or thiourea.

**6.** The process according to claim 3 or 4, wherein the organic solvent is selected from C₁-C₅ alcohols, and mixtures thereof.

**7.** The process according to claim 6, wherein the organic solvent is selected from among methanol, ethanol and/or isopropanol and mixtures thereof.

**8.** The process according to claim 3, wherein the procedure of recovering comprises the steps of:
(a) optionally cooling the reaction mixture to precipitate side products
(b) adding an organic solvent, exhibiting a polarity lower that the solvent used during the reaction and mixing;
(c) optionally adding a base;
(d) separating the organic phase containing memantine, and optionally adding hydrochloric acid to form the hydrochloric salt of memantine;
(e) precipitating memantine or its hydrochloric salt from the organic phase; and
(f) drying the resulting product.

**9.** The process according to any of the claims 3 to 8, wherein the organic solvent is selected from the group consisting of acetates, preferably from ethylacetate, isopropylacetate, butylacetate acetate; ethers, preferably from diethylether, t-butylmethyl ether or diisopropylether; alcohols, preferably linear or branched C₁-C₈ alcohols, ketones, toluene and methylenchloride, and mixtures thereof.

**10.** The process according to claim 8 or 9, wherein the organic solvent in step (b) is isopropylacetate.

**11.** The process according to any of the claims 3 to 10, further comprising the step of a recrystalization of the dried memantine hydrochloride acid salt.

**12.** A process for preparing memantine and/or its hydrochloride acid salt form comprising the steps of reacting of a compound Formula III, wherein X is Br, Cl or OH group, to obtain memantine and/or its hydrochloride acid salt form having less than 0.15 area % of 1-acetamido-3,5-dimethyladamantane.

**13.** The process according to claim 12, wherein the area is less than 0.10 %.

**14.** The process according to claim 12, wherein the area is less than 0.05 %

**15.** A pharmaceutical composition comprising memantine and/or its hydrochloride acid salt form as obtained in claim 12, wherein the pharmaceutical composition is a solid dosage form.

**16.** A pharmaceutical composition according to claim 1, wherein the solid dosage form is a film coated tablets or orally disintegrating tablet.
